**Europäisches Patentamt**

**European Patent Office**

**Office européen des brevets**

(19)

(11) Veröffentlichungsnummer : **0 332 943 B1**

# EUROPÄISCHE PATENTSCHRIFT

(12)

(45) Veröffentlichungstag der Patentschrift :
09.09.92 Patentblatt 92/37

(51) Int. Cl.$^5$ : **A61M 37/00**

(21) Anmeldenummer : **89103648.5**

(22) Anmeldetag : **02.03.89**

(54) **Implantierbare Kathetervorrichtung.**

Teilanmeldung 91119240.9 eingereicht am
16/02/89.

(30) Priorität : **16.03.88 DE 3808687**

(43) Veröffentlichungstag der Anmeldung :
**20.09.89 Patentblatt 89/38**

(45) Bekanntmachung des Hinweises auf die
Patenterteilung :
**09.09.92 Patentblatt 92/37**

(84) Benannte Vertragsstaaten :
**AT BE CH DE ES FR GB IT LI NL SE**

(56) Entgegenhaltungen :
**DE-A- 3 628 337**
**DE-U- 8 810 622**
**FR-A- 2 586 569**

(73) Patentinhaber : **B. Braun Melsungen AG**
**Carl-Braun Strasse**
**W-3508 Melsungen (DE)**

(72) Erfinder : **Lehnhardt, Fritz-Joachim, Dr.**
**Eichenweg 11**
**W-3509 Malsfled (DE)**
Erfinder : **Fuchs, Jürgen**
**Wolfhager Strasse 45**
**W-3501 Emstal-Sand (DE)**

(74) Vertreter : **Selting, Günther, Dipl.-Ing. et al**
**Patentanwälte von Kreisler, Selting, Werner**
**Deichmannhaus am Hauptbahnhof**
**W-5000 Köln 1 (DE)**

**Beschreibung**

Die Erfindung bezieht sich auf eine implantierbare Kathetervorrichtung, insbesondere für die experimentelle Mikrochirurgie, gemäß dem Oberbegriff des Patentanspruches 1.

Eine implantierbare Kathetervorrichtung mit den Merkmalen des Oberbegriffs des Patentanspruchs 1 ist aus DE-OS 36 28 337 bekannt. Dabei ist das Gehäuse als flache kreisförmige Kapsel ausgebildet, von deren Umfangswand der Anschlußteil für den Katheter als radial gerichteter Anschlußstutzen seitlich nach außen abgeht. Ein Außengewindeschaft des Druckstückes ist in ein Innengewinde des Anschlußstutzens einschraubbar, um durch axiales Anpressen einer Stirnfläche gegen eine Stirnfläche des als rohrförmiger zylindrischer Ring gestalteten Klemmstückes aus Elastomermaterial, dieses radial zu deformieren und den Katheter in dem Anschlußstutzen durch eine Quetschverbindung zu fixieren und abzudichten. Die mit einer Nadel perforierbare Oberwand der Kapsel dient zur Füllung ihres Hohlraumes mit Hilfe einer Stahlkanüle eines Flüssigkeitsüberleitungssystems, z.B. einer Spritze. Zu diesem Zweck ist es notwendig, die Kapsel so zu implantieren, daß ihre Oberwand etwa parallel zu der Haut verläuft. Für die klinische Anwendung beim Menschen und auch für die experimentelle Chirurgie an größeren Versuchstieren, wie Schweinen und Hunden, ist die bekannte implantierbare Kathetervorrichtung gut anwendbar. Zur Schaffung eines dauerhaften Zuganges zum venösen und arteriellen Gefäßsystem für chronische experimentelle Studien an tierschutzgerecht kleinstmöglichen Tieren, z.B. Ratten, ist die bekannte Kathetervorrichtung jedoch als Implantat nicht geeignet, weil bei den sehr klein dimensionierten anatomischen Gegebenheiten bei dieser Tierart die Kapsel mit dem radial gerichteten Anschlußstutzen, der von einem dicken Drehknopf über den Außenumfang des Kapselkörpers hinaus verlängert wird, zu groß ist.

Das Herausführen des distalen Endes eines unterschiedlich lang subkutan getunnelten Katheters aus der Haut ist inbesondere bei sehr kleinen Tieren von jeher problematisch wegen der notwendigen sorgfältigen Pflege des Katheters für Langzeitinfusionen durch sterile Verbände, lokale und systemische Antibiotikabehandlung sowie die Verhinderung von Thrombosierungen. Unsaubere Wundverhältnisse mit lokalen Eiterungen sind häufig; die Einheilung der Leitungen in die Haut ist nicht zu friedenstellend. Bei Versuchstieren kommen als weitere Komplikationen eines aus der Haut herausgeführten Katheters das Abbeißen des freien Katheterendes, das vollständige Herausziehen sowie das Verdrehen des angeschlossenen Katheters durch Kreisbewegungen der Tiere im Käfig bis zum totalen Verschluß des Katheters hinzu. In Ermangelung eines geeigneten implantierbaren Zuspritzports für Kleinsttiere wird seit einigen Jahren in der Praxis das distale Ende eines implantierten Katheters in eine subkutane Tasche verlagert, aus der es bei Bedarf an angeschlungenen Seidenfäden nach außen hervorgezogen wird. Auch dieses Vorgehen ist aber unzureichend, weil Komplikationen durch Thrombenbildung und Infektionen nicht vermieden werden können. Die primäre Operationsmortalität bei den bekannten Methoden liegt bei bis zu 20 %. Die Versuchsperioden sind jeweils auf ca. 4 Wochen begrenzt. Die Versagerquote ist verhältnismäßig hoch.

EP-A-309 092, die gemäß Artikel 54(3) EPÜ als Stand der Technik gilt, beschreibt eine implantierbare Kathetervorrichtung mit einem kegelstumpfförmig gestalteten Gehäuse. An dem Gehäuseende kleineren Durchmessers ist eine Öffnung zur Aufnahme eines Anschlußstückes für einen Katheterschlauch ausgebildet und das Gehäuseende größeren Durchmessers weist eine von einer Kanüle durchstechbare Membran auf. Die Vorrichtung wird subkutan implantiert; dabei liegt eine innere Umfangsseite des kegelförmigen Gehäuses etwa ebenflächig auf dem Muskelgewebe auf und ihre gegenüberliegende, äußere Umfangsseite verläuft zu dem Muskelgewebe in einem spitzen Winkel. Diese Orientierung bewirkt das Entstehen eines erhöhten Hautvorsprunges im Bereich des Endes größeren Durchmessers des Gehäuses. Außerdem ergibt sich eine Abknickung des Katheterschlauches an seiner Anschlußstelle am auslaßseitigen Ende des Gehäuses. Beide Faktoren sind für die Verwendung dieser Kathetervorrichtung bei einem Kleinstversuchstier, z.B. Ratten, Meerschweinchen, Kaninchen und anderen Tieren, bei denen periphere Venen nur sehr begrenzt zur Verfügung stehen, ungünstig, denn der Hautvorsprung quält das Tier, wenn es sich bewegt und irgendwo anstößt und der abgeknickte Katheterschlauch kann zu einer Durchflußblockierung führen, die das Ergebnis des Experimentes, für das das Tier bestimmt ist, in Frage stellen kann.

Der Erfindung liegt die Aufgabe zugrunde, eine implantierbare Kathetervorrichtung der eingangs erwähnten Art so auszubilden, daß sie zum Katheterismus bei der kleinstmöglichen Versuchstierart problemlos geeignet ist.

Diese Aufgabe wird erfindungsgemäß durch die Merkmale des Patentanspruches 1 gelöst.

Auf diese Weise entsteht eine Kathetervorrichtung sehr kleiner Abmessungen, die sich in Erfüllung der Tierschutzforderung nach kleinstmöglicher Versuchstierart problemlos in Ratten, Meerschweinchen, Kaninchen und auch in Tiere, bei denen periphere Venen nur sehr begrenzt zur Verfügung stehen, wie dem Göttinger Miniaturschwein, implantieren läßt. Die ein implantierbares Miniportsystem darstellende Kathetervorrichtung ist für Kurz- oder Langzeitinfusionen für pharmakologisch-toxikologische Studien oder experimentellchirurgische

2

Anwendungen geeignet. Auch erlaubt sie streßfreie venöse oder arterielle Blutentnahmen am nicht immobilisierten Tier.

Die rohrförmige gerade Hülse wird subkutan so implantiert, daß ihre Längsachse zu der Hautoberfläche parallel verläuft und die Oberwand quer gerichtet ist. Dies bereitet keine technischen Schwierigkeiten. Nach Einheilung der Hülse mit dem an diese angeschlossenen Katheter wird z.B. zur Flüssigkeitseinleitung die Hülse mit der linken Hand unter Bildung einer Hautfalte erfaßt, und eine hohle Stahlkanüle, die mit einer Injektionsspritze oder mit einem flexiblen Überleitungssystem, an das eine Infusionspumpe angeschlossen sein kann, verbunden ist, wird mit der rechten Hand in horizontaler Richtung durch die Haut und die elastomere Oberwand der Hülse gestochen. Diese einer subkutanen Injektion entsprechende einfache Handhabung kann ohne Dichtigkeitsverlust der Vorrichtung mehrfach wiederholt werden. Der Hohlraum der Hülse und das Kathetervolumen werden vor der Implantation und während der gesamten Untersuchungsdauer periodisch mit Heparinlösung gefüllt; das Heparindepot verhindert Thrombosierungen (Thrombophlebitiden, Thrombosen) und Ventilbildung durch flottierende Thromben sowohl bei der Implantation als auch in der Anwendungsphase. Die gute Einheilung des Implantates verhindert Infektionen. Die primäre Operationsmortalität und die Versagerquote ist gering.

Die Anwendung der erfindungsgemäßen Kathetervorrichtung für Kleinsttiere wird durch die lecksichere lösbare Quetschverbindung des Katheters mit der Hülse vorteilhaft ergänzt. Es kann bei bereits in das Gefäßsystem des Versuchstieres implantierter und genau lokalisierter Katheterspitze die Hülse an einer geeigneten Stelle in der Unterhaut des Versuchstieres plaziert, die erforderliche Katheterlänge abgeschnitten und die Quetschverbindung zwischen Katheterende und dem Anschlußteil der Hülse hergestellt werden. Hierdurch ist es möglich, bestimmten anatomischen Gegebenheiten des jeweiligen Versuchstieres bei der Implantation Rechnung zu tragen. Alternativ kann zunächst die Hülse einer vollständig vormontierten Kathetervorrichtung an ihrem Implantationsort fixiert, dann die Katheterspitze auf die zur Erreichung der Zielstelle erforderliche Länge geschnitten und dann intravasal implantiert werden.

Vorteilhafterweise besteht der Katheter aus Silicon. Ein solcher Katheter ist weich. Seine Weichheit verhindert Gefäßwandschäden oder Perforationen. Das Material ist gut antithrombogen. Im Gegensatz zu PVC verhärtet Silicon auch bei länger dauernden Inkorporationen nicht, so daß auch keine späten Komplikationen zu erwarten sind. In großen Gefäßen kommt es nicht zu übermäßiger Thrombenbildung. Der weiche Siliconkatheter läßt sich in Anpassung an die kleine Dimensionierung der Hülse so klein bemessen, daß er auch in die Arteria carotis oder femoralis einer Ratte implantiert werden kann und Zugang zum Gefäßsystem verschafft. Eine Lumenverengung durch Biegung oder Knickung des weichen Katheters am Austritt aus dem Druckstück verhindert der Knickschutzschlauch. Dieser besteht vorzugsweise aus einem Schlauchabschnitt aus Polyurethan.

Vorteilhafterweise ist der Hohlraum mit dem Klemmraum des Anschlußteiles durch einen Kanal verbunden, in dem ein Ende einer starren Stützkanüle zur inneren Abstützung des Endabschnittes des Katheters befestigt ist. Die starre Stützkanüle in dem Katheter verhindert, daß bei Deformation des elastomeren Klemmstückes unter dem Einfluß des Druckstückes das Lumen des weichen Siliconkatheters zusammengequetscht wird. Ohne besondere Vorsichtsmaßnahme kann das Druckstück fest gegen das elastomere Klemmstück angepreßt werden, damit der Katheter zwischen der starren Stützkanüle und dem elastomeren Klemmstück dicht und fest eingeklemmt gehalten ist. Die Stützkanüle ist an ihrer Spitze stumpf. Eine Beschädigung des Katheters durch die Stützkanüle wird im übrigen dadurch verhindert, daß das Druckstück die stumpfe Spitze der Stützkanüle um mehrere Millimeter nach außen überragt. Die Abmessungen der Stützkanüle ermöglichen bei kleinstmöglicher Hülsengröße eine ausreichende Infusionsgeschwindigkeit bis etwa 1 ml/sec. bei entsprechendem Druck und vermitteln damit auch große Infusionsvolumina.

Zweckmäßigerweise ist das elastomere Klemmstück hutförmig ausgebildet und der Klemmraum dem Klemmstück angepaßt gestaltet. Das Druckstück ist mit einem Außengewindeschaft in ein Innengewinde des Anschlußteiles einschraubbar und drückt mit einer ebenen Stirnfläche gegen eine ebene Fläche des Teiles vergrößerten Durchmessers des Klemmstückes. Diese Ausbildung hat sich bei der Miniaturhülse hinsichtlich eines günstigen Kraftlinienverlaufes zur radialen Einwärtsverformung des elastomeren Klemmstückes als günstig erwiesen.

An dem Außengewindeschaft des Druckstückes ist zweckmäßigerweise ein koaxialer Rohrteil mit einem Griffring vorgesehen, dessen Außendurchmesser höchstens dem Außendurchmesser des Anschlußteiles der Hülse entspricht. Das rohrförmige Druckstück setzt die Hülse koaxial fort, ohne ihren Außendurchmesser zu vergrößern. Auch hierdurch ist die Hülse zur Implantation bei Kleinsttieren besonders geeignet.

Eine Fixierung der Hülse auf der Unterlage ist erforderlichenfalls durch eine Ligatur mit einem nicht resorbierbaren monofilen Faden möglich, der in mindestens eine auf der Außenfläche der Hülse vorgesehene Ringnut eingelegt ist. Die Ringnut kann sich zwischen einer umfangsmäßigen Ringwulst der Hülse und einem an einem Ende der Hülse befestigten Haltering für eine die Oberwand bildende Membran befinden. Die Membran besteht aus einem perforierbaren Siliconstopfen, der Eigenschaften besitzt, die bei vielfacher Punktion mit Stahlkanülen eine absolute Dichtigkeit der Oberwand des Hohlraumes gewährleisten.

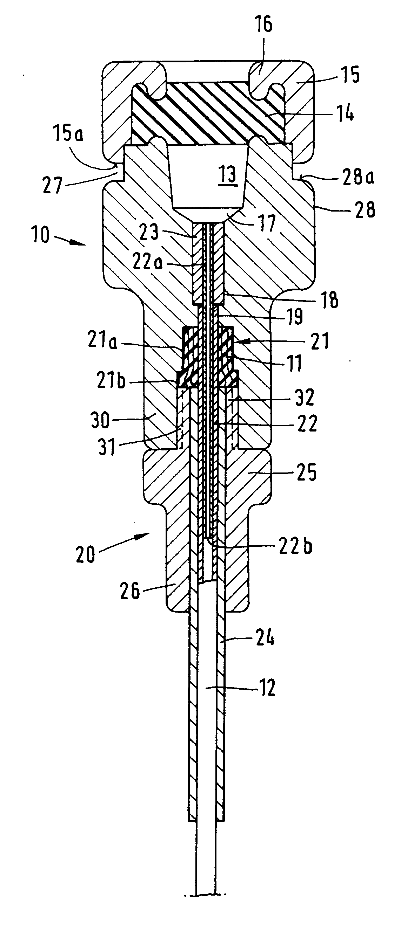

In der Zeichnung ist ein Ausführungsbeispiel der Erfindung schematisch dargestellt.

Die einzige Figur zeigt einen Längsschnitt durch eine implantierbare Kathetervorrichtung für Kleinsttiere.

Die in der nachfolgenden Beschreibung angegebenen Maße einzelner Teile sind auf ein z.B. für Ratten passendes Beispiel abgestimmt.

Die implantierbare Kathetervorrichtung besteht aus einer rohrförmigen, geraden Hülse 10 mit kreiszylindrischem Querschnitt, deren eines rohrförmiges Ende als Anschlußteil 30 ausgebildet ist, der auf dem äußeren Teil seiner Länge ein Innengewinde 31 aufweist. In das Innengewinde 31 des Anschlußteiles 30 ist ein Außengewindeschaft 32 eines rohrförmigen Druckstückes 20 mit kreiszylindrischem Innen- und Außendurchmesser zur Befestigung eines Katheters 12 aus Silicon (200 x 0,6 x 1,0 mm) einschraubbar, wie nachfolgend erläutert wird. Die Abmessungen von verschlossener Hülse 10 mit Druckstück 20 betragen etwa 25 x 10 mm ⌀.

An dem dem Anschlußteil 30 koaxial gegenüberliegenden Ende der Hülse 10 ist ein zentraler, konisch nach außen erweiterter Hohlraum 13 ausgebildet, der einen schrägen inneren Boden 17 und eine von einer durchstechbaren Oberwand 14 verschlossene äußere Öffnung besitzt. Die Oberwand 14 besteht aus einem Siliconstopfen, der von einem kreisförmigen Haltering 15 fixiert ist, welcher fest mit dem Ende der Hülse 10 verschweißt ist. Ein wulstförmiger Kragen 16 begrenzt die Außenfläche der Oberwand 14, wodurch das Auffinden die Oberwand 14 und ihr Durchstechen mit einer Stahlkanüle durch die Haut eines Versuchstieres hindurch erleichtert wird. Ausreichend für Kleinsttiere ist ein Volumen von 0,2 ml des Hohlraumes 13. In den schrägen Boden 17 des Hohlraumes 13 mündet ein zylindrischer, konzentrischer Kanal 18, an den sich koaxial ein verengter zylindrischer Kanalabschnitt 19 anschließt, der seinerseits in einen zylindrischen Klemmraum 21 zur Aufnahme eines hutförmigen Klemmstückes 11 aus Elastomermaterial mündet. Der Klemmraum 21 befindet sich in dem Anschlußteil 30 am inneren Ende des Innengewindes 31; er ist zweifach abgestuft und besteht aus einem inneren längeren Abschnitt 21a kleineren Durchmessers und einem kürzeren Abschnitt 21b etwas größeren Durchmessers. In diese beiden Abschnitte 21a und 21b des Klemmraumes 21 ist das passend hutförmig profilierte Klemmstück 11 eingesetzt, das von einem zentralen Durchlaß durchsetzt ist.

Der Kanal 18 der Hülse 10 dient der Befestigung eines Endes 22a einer starren Stützkanüle 22 aus Metall mit Hilfe einer Preßbuchse 23. Die Stützkanüle 22 ragt über die anschlußteilseitige Stirnfläche der Hülse 10 hinaus und endet in einer stumpfen Spitze 22b.

In dem axialen Durchlaß des rohrförmigen Druckstückes 20 ist ein Knickschutzschlauch 24 aus Polyurethan befestigt, der ca. 10 mm lang ist. Das eine Ende des Knickschutzschlauches 24 schließt bündig mit der Stirnfläche des Außengewindeschaftes 32 des Druckstückes 20 ab. Sein anderes Ende steht über die äußere Stirnfläche des Druckstückes 20 ca. 5 mm vor, um einen Abschnitt des Katheters 12 gegen Knickung zu schützen. An dem Druckstück 20 ist zwischen seinem Außengewindeschaft 32 und einem koaxialen Rohrteil 26 ein Griffring 25 ausgebildet, dessen Außendurchmesser dem Außendurchmesser der Hülse 10 im wesentlichen entspricht.

Zur Befestigung des Katheters 12 an der Hülse 10 wird zunächst das Ende des Katheters 12 durch den Knickschutzschlauch 24 des Druckstückes 20 hindurchgeschoben, bis es über die Stirnfläche des Außengewindeschaftes 32 ein Stück übersteht. Sodann wird der Katheter 12 auf die über die Stirnfläche des Anschlußteiles 30 hinausragende stumpfe Spitze 22b der Stützkanüle 22 aufgesteckt und durch den Durchlaß in dem Klemmstück 11 hindurch bis zur Anlage gegen die Stirnfläche der Preßbuchse 23 vorgeschoben. Anschließend wird das Druckstück 20 an seinem Griffring 25 erfaßt und es wird der Außengewindeschaft 32 vorsichtig und langsam in den Anschlußteil 30 hinein- geschraubt. Die Stirnfläche des Außengewindeschaftes 32 preßt dabei gegen die Stirnfläche des Klemmstückes 11. Dieses wird axial zusammengequetscht und deformiert sich radial, so daß der Katheter 12 zwischen der Innenfläche des Durchganges des Klemmstückes 11 und der Außenfläche der Stützkanüle 22 eingeklemmt wird. Dies hat sowohl eine Fixierung des Katheters 12 in der Hülse 10 als auch seine Abdichtung zu dem Anschlußteil 30 zur Folge. Eine Beschädigung des Katheters 12 durch die Stützkanüle wird dadurch verhindert, daß der Rohrteil 26 des Druckstückes 20 die stumpfe Spitze 22b der Stützkanüle 22 um ca. 4 mm überragt.

Wenn die Kathetervorrichtung im Empfänger fixiert werden muß, wird in eine Ringnut 27 im Bereich der Membran 14 eine Ligatur mit einem nicht resorbierbaren Nahtmaterial gelegt. Die Ringnut 27 befindet sich zwischen einer Schulter 28a einer umfangsmäßigen Ringwulst 28 an dem hohlraumseitigen Ende der Hülse 10 und einer Schulter 15a des Halteringes 15.

Vor der Implantation der Kathetervorrichtung werden mit einer durch die Oberwand 14 hindurchgestochenen Stahlkanüle der Hohlraum 13 und das Lumen des Katheters 12 mit heparinisierter Kochsalzlösung gefüllt und die Dichtigkeit des Anschlusses an dem Klemmstück 11 wird geprüft.

EP 0 332 943 B1

## Patentansprüche

1. Implantierbare Kathetervorrichtung für die experimentelle Mikrochirurgie, bestehend aus einem Gehäuse, das einen Hohlraum (13), eine Umfangswand und eine mit einer Nadel durchstechbare Oberwand (14) aufweist, einem von der Umfangswand abgehenden hohlen Anschlußteil (30), einem in den hohlen Anschlußteil (30) einschiebbaren Katheter (12), und einer Befestigungsanordnung (11,20) für den Katheter (12), die zusammengesetzt ist aus einem elastomeren Klemmstück (11), das den Katheter (12) in einem Klemmraum (21) des Anschlußteiles (30) umgibt, und einem mit dem Anschlußteil (30) verbindbaren Druckstück (20), durch das der Katheter (12) verläuft und das axial gegen das Klemmstück (11) drückt, um es durch radiale Verformung abdichtend gegen den Katheter (12) anzupressen,
**dadurch gekennzeichnet,**
daß das Gehäuse als rohrförmige gerade Hülse (10) ausgebildet ist, deren einer Endbereich den Anschlußteil (30) bildet und deren anderer Endbereich den Hohlraum (13) enthält, dessen durchstechbare Oberwand (14) an der Stirnseite der Hülse (10) angeordnet ist und daß in dem Druckstück (20) ein Ende eines den Katheter (12) auf einem Teil seiner Länge umgebenden Knickschutzschlauches (24) befestigt ist.

2. Implantierbare Kathetervorrichtung nach Anspruch 1, dadurch gekennzeichnet, daß der Hohlraum (13) mit dem Klemmraum (21) des Anschlußteiles (30) durch einen Kanal (18,19) verbunden ist, in dem ein Ende einer starren Stützkanüle (22) zur inneren Abstützung des Endabschnittes des Katheters (12) befestigt ist.

3. Implantierbare Kathetervorrichtung nach Anspruch 2, dadurch gekennzeichnet, daß die Stützkanüle (22) mit einer Preßbuchse (23) in dem Kanal (18, 19) befestigt ist und daß die Stirnfläche des Katheters (12) an die ihr zugewandte Stirnfläche der Preßbuchse (23) anstößt.

4. Implantierbare Kathetervorrichtung nach einem der Ansprüche 1 bis 3, dadurch gekennzeichnet, daß das elastomere Klemmstück (11) hutförmig ausgebildet und der Klemmraum (21) dem Klemmstück (11) angepaßt gestaltet ist.

5. Implantierbare Kathetervorrichtung nach einem der Ansprüche 1 bis 4, dadurch gekennzeichnet, daß das Druckstück (20) mit einem Außengewindeschaft (32) in ein Innengewinde (31) des Anschlußteiles (30) einschraubbar ist und mit einer ebenen Stirnfläche gegen eine ebene Fläche des Teiles vergrößerten Durchmessers des Klemmstückes (11) drückt.

6. Implantierbare Kathetervorrichtung nach Anspruch 5, dadurch gekennzeichnet, daß an dem Außengewindeschaft (32) des Druckstückes (20) ein koaxialer Rohrteil (26) mit einem Griffring (25) vorgesehen ist, dessen Außendurchmesser höchstens gleich dem Außendurchmesser des Anschlußteiles (30) der Hülse (10) ist.

7. Implantierbare Kathetervorrichtung nach einem der Ansprüche 1 bis 6, dadurch gekennzeichnet, daß auf der Außenfläche der Hülse (10) mindestens eine Ringnut (27) ausgebildet ist.

8. Implantierbare Kathetervorrichtung nach Anspruch 7, dadurch gekennzeichnet, daß die Ringnut (27) sich zwischen einer umfangsmäßigen Ringwulst (28) der Hülse (10) und einem an einem Ende der Hülse (10) befestigten Haltering (15) für eine die Oberwand (14) bildende Membran befindet.

9. Implantierbare Kathetervorrichtung nach einem der Ansprüche 1 bis 8, dadurch gekennzeichnet, daß der Katheter (12) aus Silicon besteht.

10. Implantierbare Kathetervorrichtung nach einem der Ansprüche 1 bis 9, dadurch gekennzeichnet, daß der Haltering (15), die Hülse (10) und das Druckstück (20) kreiszylindrischen Außendurchmesser aufweisen.


## Claims

1. Implantable catheter device for experimental microsurgery, consisting of a housing with a cavity (13), a circumferential wall and an upper wall (14) being pierceable with a needle, a hollow connector (30) branching from said circumferential wall, a catheter (12) insertable into said hollow connector (30), and a

5

fastening assembly (11, 20) for said catheter (12), which is composed of an elastomeric clamping member (11) enclosing said catheter (12) within a clamping chamber (21) of said connector (30), and a pressure member (20) connectable with said connector (30), said pressure member being traversed by said catheter (12) and pressing axially against said clamping member (11) in order to press the same against said catheter (12) in a sealing manner by radial deformation, characterised in that said housing is formed as a straight tubular sleeve (10), one end portion of which forms said connector (30) and the other end portion of which includes said cavity (13), the pierceable upper wall (14) of which being provided at the front end of said sleeve (10), and in that one end of an antikink hose (24), surrounding said catheter (12) over a part of the length thereof, is fastened in said pressure member (20).

2. Implantable catheter device of claim 1, characterised in that said cavity (13) is connected with said clamping chamber (21) of said connector (30) via a channel (18, 19) in which an end of a rigid supporting cannula (22) is fastened for internally supporting said end portion of said catheter (12).

3. Implantable catheter device of claim 2, characterised in that said supporting cannula (22) is fastened in said channel (18, 19) by means of a press bushing (23), and in that the front end surface of said catheter (12) abuts the confronting front end surface of said press bushing (23).

4. Implantable catheter device of one of claims 1 to 3, characterised in that said elastomeric clamping member (11) is hat-shaped and said clamping chamber (21) is shaped in conformity with said clamping member (11).

5. Implantable catheter device of one of claims 1 to 4, characterised in that said pressure member (20) is screwable with an externally threaded shaft (32) into an internal thread (31) of said connector (30) and presses with a planar front end surface against a planar surface of the increased diameter portion of said clamping member (11).

6. Implantable catheter device of claim 5, characterised in that a coaxial tube portion (26) with a handle ring (25) is provided at said externally threaded shaft (32) of said pressure member (20), the outer diameter of said tube portion being at most equal to the outer diameter of said connector (30) of said sleeve (10).

7. Implantable catheter device of one of claims 1 to 6, characterised in that at least one annular groove (27) is formed on the outer surface of said sleeve (10).

8. Implantable catheter device of claim 7, characterised in that said annular groove (27) is arranged between a circumferential annular bead (28) of said sleeve (10) and a holding ring (15), fastened at one end of said sleeve (10), for a diaphragm forming said upper wall (14).

9. Implantable catheter device of one of claims 1 to 8, characterised in that said catheter (12) consists of silicone.

10. Implantable catheter device of one of claims 1 to 9, characterised in that said holding ring (15), said sleeve (10) and said pressure member (20) have a circular cylindrical outer diameter.

## Revendications

1. Dispositif de cathéter implantable destiné à la microchirurgie expérimentale, se composant d'un boîtier, qui présente un espace creux (13), une paroi périphérique et une paroi supérieure (14) perçable avec une aiguille, une partie de raccordement creuse partant de la paroi périphérique, un cathéter (12) insérable dans la partie de raccordement creuse (30), et d'un agencement de fixation (11, 20) pour le cathéter (12), qui est composé d'un élément de serrage élastomère (11), qui entoure le cathéter (12) dans un espace de serrage (21) de la partie de raccordement (30), et d'un élément de pression (20) à travers lequel passe le cathéter (12) qui peut être relié avec la partie de raccordement (30) et pousse axialement contre l'élément de serrage (11), afin de le presser contre le cathéter (12) de façon étanche grâce à une déformation radiale, caractérisé en ce que

le boîtier est réalisé sous forme de douille droite tubulaire (10), dont une première zone d'extrémité constitue la partie de raccordement (30) et dont l'autre zone d'extrémité contient l'espace creux (13) dont la paroi supérieure (14) perçable est agencée au côté frontal de la douille (10) et en ce qu'une extrémité d'un tube souple de protection contre le flambage (24), entourant le cathéter (12) sur une partie de sa longueur, est fixée dans l'élément de pression (20).

2. Dispositif de cathéter implantable selon la revendication 1, caractérisé en ce que l'espace creux (13) est relié avec l'espace de serrage (21) de la partie de raccordement (30) par un canal (18, 19), dans lequel une extrémité d'une canule de support rigide (22) est fixée en vue de supporter à l'intérieur la section d'extrémité du cathéter (12).

3. Dispositif de cathéter implantable selon la revendication 2, caractérisé en ce que la canule de support (22) est fixée avec une douille de pression (23) dans le canal (18, 19) et en ce que la surface frontale du cathéter (12) vient heurter la surface frontale de la douille de pression (23) qui est tournée vers elle.

4. Dispositif de cathéter implantable selon l'une des revendications 1 à 3, caractérisé en ce que l'élément de serrage élastomère (11) est en forme de chapeau et en ce que l'espace de serrage (21) est d'une forme adaptée à l'élément de serrage (11).

5. Dispositif de cathéter implantable selon l'une des revendications 1 à 4, caractérisé en ce que l'élément de pression (20) peut être vissé, au moyen d'une tige à filet extérieur (32), dans un filet intérieur (31) de la partie de raccordement (30) et qu'il pousse avec une surface frontale plane contre une surface plane de la partie de plus grand diamètre de l'élément de serrage (11).

6. Dispositif de cathéter implantable selon la revendication 5, caractérisé en ce qu'il est prévu à la tige à filet extérieur (32) de l'élément de pression (20) une partie tubulaire coaxiale (26) pourvue d'une bague de saisie (25) dont le diamètre extérieur est au plus égal au diamètre extérieur de la partie de raccordement (30) de la douille (10).

7. Dispositif de cathéter implantable selon l'une des revendications 1 à 6, caractérisé en ce qu'au moins une gorge annulaire (27) est formée sur la surface extérieure de la douille (10).

8. Dispositif de cathéter implantable selon la revendication 7, caractérisé en ce que la gorge annulaire (27) se trouve entre un bourrelet annulaire périphérique (28) de la douille (10) et une bague de maintien (15) pour une membrane constituant la paroi supérieure (14), qui est fixée à une extrémité de la douille (10).

9. Dispositif de cathéter implantable selon l'une des revendications 1 à 8, caractérisé en ce que le cathéter (12) est composé de silicone.

10. Dispositif de cathéter implantable selon l'une des revendications 1 à 9, caractérisé en ce que la bague de maintien (15), la douille (10) et l'élément de pression (20) présentent des diamètres extérieurs cylindriques circulaires.